# EUROPEAN PATENT SPECIFICATION

(11) **EP 4 157 419 B1**
(45) Date of publication and mention of the grant of the patent: **13.08.2025**
(21) Application number: 21737232.5
(22) Date of filing: 25.05.2021
(51) Int. Cl.: A61M 25/00

(54) **ONE-PIECE INTERMITTENT CATHETERS WITH VARIABLE THICKNESS AND HANDLE FEATURE**
EINTEILIGE INTERMITTIERENDE KATHETER MIT VARIABLER DICKE UND GRIFFFUNKTION
CATHÉTERS INTERMITTENTS MONOBLOCS À ÉPAISSEUR VARIABLE ET CARACTÉRISTIQUE DE POIGNÉE

(30) Priority: 26.05.2020 US 202063030024 P
(43) Date of publication of application: 05.04.2023
(60) Divisional application: 25184762.0
(73) Proprietor: Hollister Incorporated, Libertyville, IL 60048 (US)
(72) Inventor: O'DONNELL, Paul M., Libertyville, IL 60048 (US); KEARNS, Barbara J., Libertyville, IL 60048 (US)
(74) Representative: FRKelly
(86) International application number: PCT/US2021/034043
(87) International publication number: WO 2021/242745

(56) References cited:
- CN-U- 203 264 007
- CN-U- 206 239 873
- US-A- 5 865 815

## Description

### TECHNICAL FIELD

The present disclosure generally relates to urinary catheters that potentially reduce the likelihood of UTls (Urinary Tract Infections), and more particularly, to urinary catheters that allow flushing of the urethra during catheterization.

### BACKGROUND

Intermittent urinary catheters are commonly used by those who suffer neurogenic conditions that affect bladder function, such as but not limited to SCI or multiple sclerosis, and for those with urinary retention. Urinary catheters drain urine from the bladder through a shaft and out a proximal end without the urine contacting any portion of the urinary tract. Because the users are unable to void directly through the urethra, the urethra no longer experiences the continuous flushing and re-wetting of the tissues that normally occurs during active voluntary urination. The first 1.5cm / 15mm of the distal urethra is densely populated with micro-organisms. The absence of flushing and rewetting may lead to an increased risk of urinary tract infections. Furthermore, although catheters are provided in sterile and contamination free state, users are at risk to contract a urinary tract infection due to contamination by, for example, the user touching the catheter before insertion, reuse of catheters, and not following proper catherization techniques.

There remains a need for catheters having a design that reduces the risk of UTIs. US5865815, CN206239873U and CN203264007U disclose three exemplary urinary catheters proposing different approaches to reduce UTI.

### SUMMARY

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto. The invention is defined in claim 1. Preferable embodiments are defined in the dependent claims.

In one aspect, a catheter includes a catheter shaft with a proximal portion and a distal portion. There is at least one eyelet in the proximal portion being in communication with an inner lumen of the proximal portion. There is at least one drainage opening in the catheter shaft for draining fluid from the inner lumen. The drainage opening is between the proximal portion and the distal portion of the catheter shaft. The distal portion includes at least one rod. The at least one drainage opening is for draining fluid from the inner lumen to the outside of the at least one rod of the catheter shaft.

### BRIEF DESCRIPTION OF DRAWINGS

Fig. 1 includes a top perspective view of a catheter according to one embodiment of the current disclosure.
Fig. 1A includes a top perspective view of a catheter according to one embodiment of the current disclosure.
Fig. 2A is a cross-sectional view of the catheter of Fig. 1 taken through line 2A-2A.
Fig. 2B is a cross-sectional view of the catheter of Fig. 1 taken through line 2B-2B.
Fig. 2C is a cross-sectional view of the catheter of Fig. 1 taken through line 2C-2C.
Fig. 3 includes a top perspective view of a catheter according to an embodiment of the current disclosure.
Fig. 4 includes a top perspective view of a catheter according to an example of the current disclosure.
Fig. 5A is a cross-sectional view of the catheter of Fig. 4 taken through line 5A-5A.
Fig. 5B is a cross-sectional view of the catheter of Fig. 4 taken through line 5B-5B.
Fig. 5C is a cross-sectional view of the catheter of Fig. 4 taken through line 5C-5C.
Fig. 6 is an enlarged section of the catheter of Fig. 4.
Fig. 7 is a partial cross-sectional view of the catheter section of Fig. 6.

### DETAILED DESCRIPTION OF THE ILLUSTRATED EMBODIMENTS

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter as defined in the accompanying claims.

The present disclosure is directed to catheters that include a catheter shaft which enables fluid to flush a portion of the urethra of the urinary tract, helping to potentailly prevent urinary tract infections. The catheter shaft has a distal portion with a proximal end having a tip that is inserted through a user's urethra and into the bladder for draining urine therefrom. The catheter shaft also includes a distal portion that supports insertion of the proximal portion and allows urine flow to contact the urethra wall during catheterization, which may assist in wetting and flushing out the urethra.

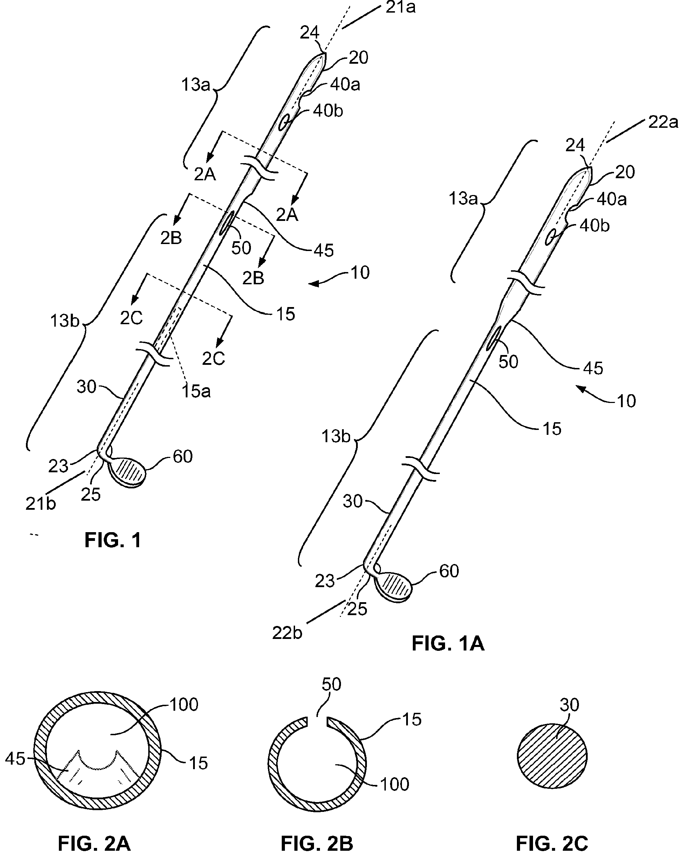

Fig. 1 illustrates a urinary catheter 10 having a catheter shaft 15 with a proximal portion 13a which has a proximal end 20 and a distal portion 13b which has a proximal end 25. The catheter shaft 15 may be made of a polymeric material. The polymeric material may include, but is not limited to, polyethylenes, polypropylenes, polyvinylchlorides, polytetrafluoroethylenes, polyvinylacetates, polystyrenes, polyesters, polyurethanes, polyamides, ethylene vinyl acetate copolymers, and combinations thereof.

The proximal end 20 of the catheter shaft has a tip that can be any appropriate shape for insertion through the urethra and into the bladder. In one embodiment, the proximal end 20 has a straight rounded shape, such as the illustrated nelaton shape. Other shapes are applicable such as curved and tapered (Coudé, Tiemann, Olive tip).

The proximal portion 13a of the catheter shaft 15 includes one or more draining holes or eyelets 40a, 40b in communication with an inner lumen 100 (Figs. 2A and 2B) of the proximal portion 13a of the catheter shaft. Fig. 2A shows a cross-sectional view of the proximal portion 13a of the catheter shaft 15 toward the proximal end 20 of the catheter shaft. As shown in this figure, the proximal portion 13a of the catheter shaft 15 includes an inner lumen 100. The at least one eyelet 40a, 40b may be for the drainage of bodily fluids therethrough and into an inner lumen 100 (Figs. 2A and 2B) of the catheter shaft. As shown in Fig. 1, in one embodiment, the proximal portion 13a can include at least two eyelets 40a and 40b. As shown in the figure, the eyelets 40a, 40b can be distally spaced from the proximal tip of the proximal end of the catheter. However, the eyelet or eyelets 40a, 40b may be closer to the proximal tip or may arranged in any effective manner. In one alternative, the eyelet 40a, 40b may be in or at the proximal end 20. The eyelet(s) 40a, 40b may be configured to be located within a bladder when the catheter is inserted to drain urine from the bladder.

The proximal portion 13a of the catheter 11 transitions at area 45 to the distal end portion 13b. The distal end portion 13b includes at least one rod 30 that may extend to the distal end 25 of the catheter shaft 15. The transition may be such that the axis of the rod is offset from the axis of the proximal end portion (Fig. 1). Alternatively, the transition my be such that the axis of the rod is aligned with the axis of the proximal end portion.

Referring to Fig. 1, the transition area 45 includes a neckdown or stepdown from the proximal portion 13a to the rod 30. Optionally, the neckdown may be a partial neckdown of one side of the catheter shaft. In one example, the neckdown extends around about half of the catheter. When this optional neckdown is present, the central axis 21b of the rod 30 may be offset from the central axis 21a of the proximal portion 13a. When the central axis of the rod is offset from the central axis of the proximal portion, the axes may be parallel to each other. In other embodiments, the axes may be at angles to one another.

Referring to Fig. 1A, optionally, the neckdown or stepdown at the transition area may be a circumferential neckdown or on that extends substantially about the circumference of the catheter. When this optional neckdown is present, the central axis 22b of the rod 30 may be aligned with the central axis 22a of the proximal portion 13a.

The catheter shaft 15 also includes at least one drainage opening 50 that drains fluid from the inner lumen 100. Fig. 2B shows a cross-sectional view of the catheter shaft 15 near transition area 45. Inner lumen 100 of catheter shaft 15 is in communication with drainage opening 50 which allows the urine to exit and flush the urethra outside rod 30 of the distal portion. The location of the drainage opening(s) 50 along the catheter shaft 15 is such that during catheterization, the drainage opening(s) 50 is located within the urethra. As such, during catheterization, urine exits drainage opening(s) 50 and flows into and through the urethra. The urine flows outside of at least one rod 30 and wets and flushes out the urethra.

In Fig. 1, the at least one drainage opening 50 is shown within the vicinity of area 45. The at least one drainage opening 50 may be below or above area 45. In one alternative, the drainage opening(s) 50 may between the proximal portion 13a and the distal portion 13b. The at least one drainage opening 50 may be about 50% down the length of the catheter shaft or equidistant between a terminal distal end 23 and a terminal proximal end 24 of the catheter shaft. In another alternative, the drainage opening(s) 50 may also be located closer to a terminal proximal end 24 of the catheter shaft 15 than to a terminal distal end 23 of the catheter shaft 15. Additionally, draining opening(s) may be located closer to a terminal distal end 23 than to a terminal proximal end 24 of the catheter shaft 15. Although one drainage opening 50 is shown in the figure, additional drainage openings may be present. They may also be configured to be located within the urethra. Multiple drainage openings can be placed in the same place or along different parts of the urethra. The catheter of the current disclosure, therefore, does not require a draining member or funnel at the distal end of the catheter shaft, unlike traditional catheter designs.

The proximal section 13a of the catheter 11 can be softer than the distal portion 13b of the catheter 11, which aids in easier catheter insertion, control and manipulation. Alternatively, the distal portion 13b of the catheter may be softer than the proximal portion 13a of the catheter 11. The varying in flexibility may be especially beneficial in a male catheter where the catheter is inserted along the curved or serpentine path of the urethra.

In Fig. 1, the catheter is shown having a single rod 30 of the distal portion 13b, which may be a solid structure, as shown in Fig. 2C, or may include an inner lumen. The rod 30 may maintain a consistent or similar outer diameter or may taper inward toward the proximal end or distal end. The cross-sectional shape of the rod may be any various shape, such as a circle, ellipsis, square, triangle, pentagon, hexagon, polygon, and other shapes. The rod can also be u-shaped.

The cross-sectional width of the proximal portion 13a and the distal portion 13b of the catheter may vary. Preferably, the cross-sectional width of the proximal portion 13a of the catheter is larger than the cross-sectional width of the distal portion 13b of the catheter and/or rod 30. The proximal portion 13a may gradually decrease in diameter or abruptly shift/step to a smaller diameter at the transition area 45 between the proximal portion 13a and the distal portion 13b. Furthermore, when the catheter 10 is a male catheter, it may have a length of about 40 cm, where the proximal portion 13a has a length between about 10 cm and about 16 cm, and the distal portion 13b has a length between about 24 cm and about 30 cm. In one embodiment, the ratio of the length of the proximal portion 13a to that of the length of distal portion 13b may be from 1: 3 to 1:1.5.

Optionally, the distal portion 13b of the catheter shaft 15 may have a gripper or handle 60. The handle 60 may aid with insertion, manipulation and/or control, and withdrawal of the catheter. The handle 60 may be solid and include indentations or raised portions for better grasping by the user. The handle 60 may also be flat. The handle can be any practical shape. In one embodiment, the handle may be circular or oval shaped. The handle may be stationary or flexibly move up and down to a desired angle.

The handle 60 may extend from the distal end 25 of the catheter shaft 15. In one embodiment, the handle 60 may extend at an angle from the catheter shaft 15. For example, the distal end 25 of the catheter shaft 15 may have a bend or curve projecting out at a desired angle before transitioning to the handle 60. The angle may be between 0° (no angle) and 90°. Preferably, the angle is between 30° and 90°. The handle may be orientated in any plane around the central catheter axis or be orientated in any plane around central axis of the angular extension.

Both the bend and the handle 60 can be effectively sized for use while inserting, manipulating and/or withdrawing the catheter. The handle is spaced at a sufficient distance from the bend to reduce the risk of urine contacting the user's hands. In one embodiment, length of catheter rod between the bend or curve and the handle may be about 2 cm. Optionally, the length may be about 1 cm to about 3 cm. The gripper or handle may have a length and/or width of about 1.5 cm. Preferably the gripper or handle has a length and/or width of about 1 cm to about 2 cm.

The method of making the catheter shown in Fig. 1 may include processing or forming a catheter tube into the above described structure. For example, the tip at the proximal end 20 and eyelet(s) 40a, 40b may be formed in the proximal portion 13a of the shaft 15. The distal portion 13b of the shaft 15 may then be crimped or necked down by a heating and compression process. For example, the distal portion 13b of the catheter shaft 15 may be placed in a tool that heats and compresses the distal portion 13b of the catheter shaft 15 into a rod 30 with or without a central lumen. Optionally, the distal portion 13b of catheter shaft 15 may include a central (core) axial mandrel/reinforcment member 15a. If present, the mandrel/reinforcement member 15a may be made from various metals and/or plastics. The mandrel/reinforcement member 15a may be for example a stainless steel or polymer spring or rod. The drainage opening 50 may be formed in the shaft 15 before or after forming the distal portion 13b.

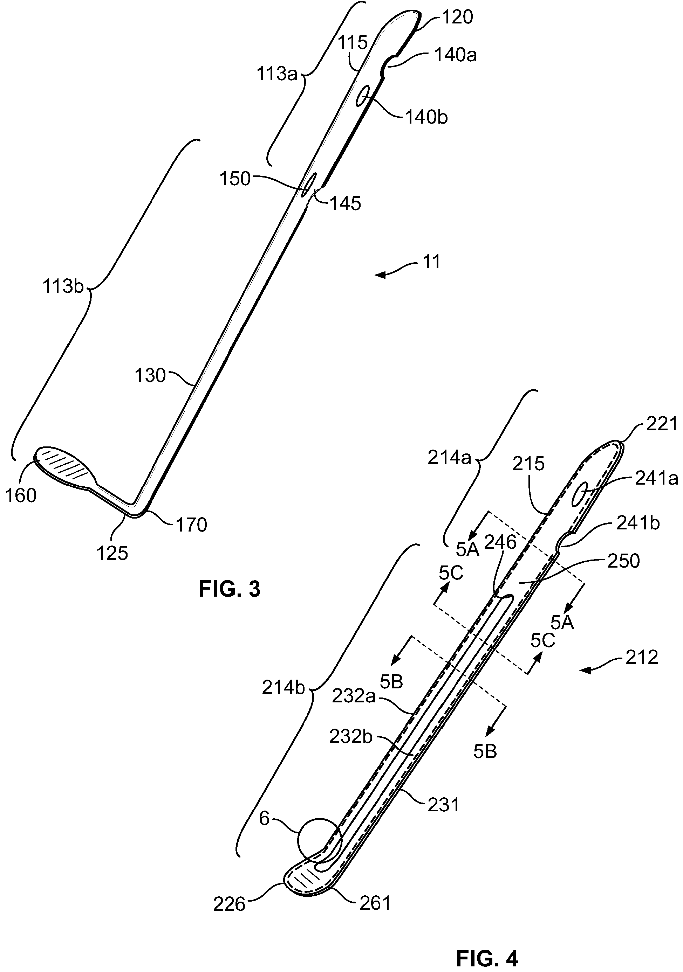

Fig. 3 illustrates another embodiment of a female catheter 111 according to the current disclosure. Catheter 111 is similar to that of catheter 10 shown in Fig. 1, in that catheter 111 has several of the same or similar features of the catheter 10. Similar features are numbered in a similar manner. Similar to catheter 10, catheter 111 has a catheter shaft 115 with a proximal portion 113a that includes a proximal end 120 and a proximal portion 113b that includes a proximal end 125.

The proximal portion 113a of the catheter shaft includes one or more draining holes or eyelets 140a, 140b in communication with an inner lumen (not shown) of the proximal portion of the catheter shaft. The at least one eyelet 140a, 140b may be for the drainage of bodily fluids therethrough and into an inner lumen of the catheter shaft.

The proximal portion 113a of the catheter shaft 115 transitions at area 145 to at least one rod 130 toward the distal portion 113b of the catheter shaft. Similar to that of Figs. 1 and 1A, the central axis of the rod 130 may be aligned with the central axis of the proximal portion 113a or the central axis of the rod 130 may be offset from the central axis of the proximal portion 113a. When the central axis of the rod is offset it may be parallel to the central axis of the proximal portion. Below, at or near area 145, at least one drainage opening 150 drains fluid from the inner lumen to flush out the urethra to the outside of the distal portion which includes at least one rod 130.

The proximal portion 113a may gradually decrease in diameter or abruptly shift/step to a smaller diameter at the transition area 145 between the proximal portion 113a and the distal end portion 113b. Furthermore, when the catheter 111 is a female catheter, it may have a catheter shaft length of about 17 cm, where the proximal portion 113a has a length between about 3.5 cm and about 5 cm, and the distal end has a length between about 12 cm and about 13.5 cm. In one embodiment, the ratio of the length of the proximal portion 113a to that of the length of distal portion 113b may be from 1:3.8 to 1:2.4.

The distal portion 113b of the catheter shaft may have a gripper or handle 160. The handle 160 may aid with insertion, manipulation and/or control, and withdrawal of the catheter.

Fig. 4 illustrates an additional example of the current disclosure, catheter 212. Catheter 212 has a catheter shaft with a proximal portion 214a that includes a proximal end 221 and a distal portion 214b that includes a distal end 226. The catheter shaft can be made of any of the polymeric material described herein.

The proximal end 221 of the catheter shaft has a tip that can be any appropriate shape, such as a rounded shape. In the illustrated example, the proximal end 221 has a nelaton shape.

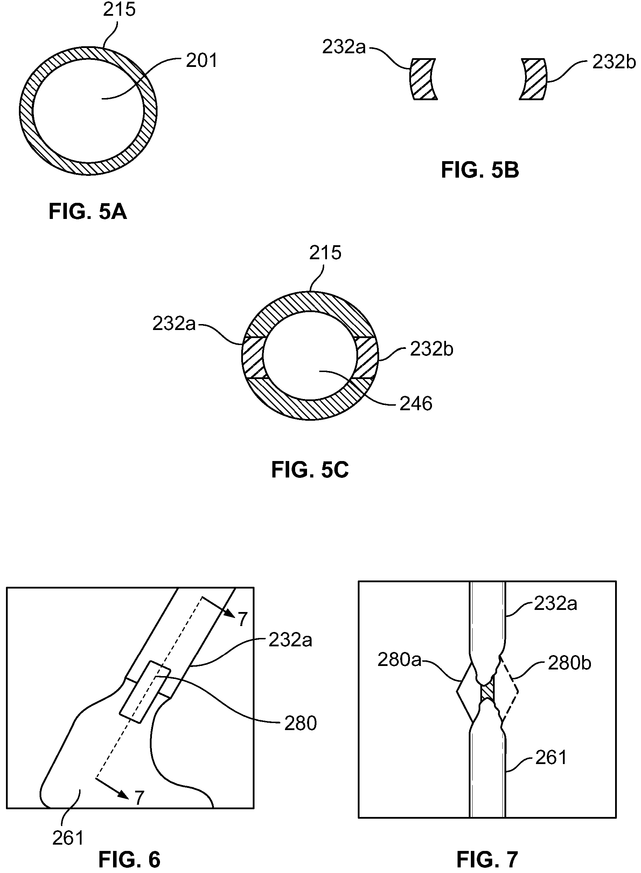

The proximal portion 214a of the catheter shaft includes one or more draining holes or eyelets 241a, 241b in communication with an inner lumen 201 of the proximal portion 214a of the catheter shaft 215. Fig. 5A shows a cross-sectional view of the proximal portion 214a of the catheter shaft 215 toward the proximal end 221 of the catheter shaft. At cross section 5A, there is an inner lumen 201. The at least one eyelet may be for the drainage of bodily fluids therethrough and into an inner lumen of the catheter shaft. As shown in Fig. 4, in one example, the proximal portion 214a can include at least two eyelets 241a and 241b. As shown in the figure, the eyelets can be distally spaced from the proximal tip of the proximal end of the catheter. However, the eyelet or eyelets can be closer to the proximal tip and arranged in any effective manner. The eyelet or eyelets may be configured to be located within a bladder when the catheter is inserted.

The proximal portion 214a of the catheter shaft 215 transitions at area 250 to the distal portion 214b. The distal portion includes at least two rods 232a and 232b that extend toward the distal end 226 of the catheter shaft 215. Fig. 5C shows a cross-section at a point on the catheter shaft near transition area 250. Below, at or near area 250, at least one drainage opening 246 drains fluid from the inner lumen to the outside of the distal portion 214b and rods 232a and 232b. The drainage opening 246 can be the end of the inner lumen or can be a side opening in the catheter shaft 215. The drainage opening 246 at transition area 250 allows the urine to exit to outside of the catheter shaft 215 and into the urethra, where the urine wets and flushes out the urethra as it flows therethrough.

Similar to the catheters of Figs. 1 and 3, the drainage opening is between the proximal portion 214a and the distal portion 214b. The at least one drainage opening 246 may be about 50% down the length of the catheter shaft or equidistant between a terminal distal end and a terminal proximal end of the catheter shaft. The drainage opening 246 may also be located closer to a terminal proximal end of the catheter shaft than to a terminal distal end of the catheter shaft. Additionally, the draining opening 246 may be located closer to a terminal distal end than to a terminal proximal end of the catheter shaft. The transition area and drainage opening are preferably configured to be located within the urethra when the catheter is inserted. Although one drainage opening 246 is shown in the figure, additional drainage openings may be present. They may also be configured to be located within the urethra. Multiple drainage openings can be placed in the same place or along different parts of the urethra. The catheter of the current disclosure, therefore, does not require a draining member or funnel at the distal end of the catheter shaft, unlike traditional catheter designs.

The rods 232a and 232b may be solid structures. The rods may maintain a consistent or similar outer diameter and may taper out to the edge. The crosssections of these rods can have various shapes, such as a circle, ellipsis, square, triangle, pentagon, hexagon, polygon, and other shapes. The rod can also be u-shaped. Cross section 5B shows the catheter shaft portion 215 including rods 232a and 232b.

The cross-sectional width of the proximal portion 214a and the distal portion 214b of the catheter may be similar or vary. Preferably, the cross-sectional width of the proximal portion 214a of the catheter is smaller than the cross-sectional width of the distal portion 214b of the catheter.

The distal portion 214b of the catheter shaft may have a gripper or handle 261. The handle 261 can be formed from the two rods 232a and 322b. The handle 261 can join the two rods 232a and 232b at the distal end of the catheter shaft.

Fig. 6 shows an enlarged view of joint between a portion of the rod 232a and gripper/handle 261 at the distal end 226 of the catheter 212. Handle 261 can have at least one piece 280 for enabling a range of movement of the gripper/handle 261. Piece 280 is attached to rod 232a and handle 261.

Fig. 7 illustrates a cross section of Fig. 6. Fig. 7 shows that there may be two pieces 280a and 280b enabling a full range of movement for handle 261 for each rod of the handle 261. Pieces 280a and 280b attach at one end to distal rod 232a and at the other end to gripper/handle 261. Rod 232b may also have a similar set of pieces. Each of pieces 280a and 280b can be composed of independent parallel arms. Each set of arms can have elbow pivot points. In one embodiment, each piece has three parallel arms, with a central arm that may be longer in length. The pieces and the associated arms allow for the handle to be moved easily from one angular position to another. The handle may also include a hinge or any other device that enables it to easily move between angular positions. The handle angle may be able to move between 0° (no angle) and 90°. Preferably, the handle is able to move between 30° and 90°.

The catheters included in the current disclosure may be formed by any known manufacturing process. The catheters may be formed in a single process or multiple processing steps. The catheters may be formed by different types of molding, more particularly by injection molding. The catheters may also be formed by heating or extruding a portion or portions to the desired shape. In a particular embodiment a formed catheter shaft is further modified by any of the above described methods to form a distal rod portion or portions.

The catheters included in the current disclosure may be, but not limited to, hydrophilic catheters and parts of the catheter shaft may include a hydrophilic coating. When the hydrophilic coating is wetted or hydrated with a hydration medium, such as water, it becomes lubricious which eases introduction of the device into the body and aids in reducing pain and discomfort associated with such introduction. The hydrophilic coating can be a single layer or multilayer hydrophilic coating. Multiple layered coating can include at least a base coat and top layer. The catheters may also include a gel on the outer surface to aid with insertion.

The catheters included in the current disclosure, optionally, also may include a thin flexible sleeve that covers at least a section of the outer surface of the catheter shaft. The sleeve may be formed of any variety of thin flexible polymeric film materials, such as polyethylene, plasticized PVC, polypropylene, polyurethane or elastomeric hydrogels. When the catheter includes a hydrophilic coating thereon, the sleeve may be liquid and/or vapor permeable so as to allow liquid and/or vapor therethrough to hydrate the hydrophilic coating while the catheter is stored within a package. Alternatively, the sleeve may include a hydration liquid or a foamed hydration liquid within the sleeve and in contact with the hydrophilic material.

Any of the above-described catheters may be used by male or female patients. Males catheters tend to differ from female catheters in length because of difference in urethra length. Female catheters of the current disclosure may be shorter overall and may also have a shorter distal end portion.

The catheters of the present disclosure may be sterilized prior to use. The catheters may be sterilized by applying a sufficient amount of radiation, such as (but not limited to) gamma or E-Beam radiation. The catheters can be sterilized with radiation while the hydrophilic coating is in contact with the wetting fluid.

It will be understood that the embodiments described above are illustrative of some of the applications of the principles of the present subject matter. Numerous modifications may be made by those skilled in the art without departing from the scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A urinary catheter (10, 111, 212) having a catheter shaft (15, 115, 215) including a distal portion (13b, 113b, 213b) and a proximal portion (13a, 113a, 213a) having a tip that is inserted through a user's urethra;
at least one eyelet (40a, 140a, 241a, 40b, 140b, 241b) in the proximal portion; the at least one eyelet being in communication with an inner lumen (100, 201) of the proximal portion to drain urine from the bladder;
at least one drainage opening (50, 150, 246) in the catheter shaft for draining fluid from the inner lumen, wherein the at least one drainage opening is between the proximal portion and the distal portion of the catheter shaft such that during catheterization, the drainage opening is configured to be located within the urethra;
the distal portion including at least one rod (30, 130, 232a, 232b); and
the at least one drainage opening for draining fluid from the inner lumen to the outside of the at least one rod of the distal portion of the catheter shaft;
**characterized in that** a cross-sectional width of the at least one rod is smaller than a cross-sectional width of the proximal portion.

2. The urinary catheter of claim 1, wherein the at least one rod is a solid structure.

3. The urinary catheter of any one of claims 1 and 2, further including a neckdown between the proximal portion and the rod.

4. The urinary catheter of claim 3, wherein the neckdown is a partial neckdown or a circumferential neckdown.

5. The urinary catheter of any one of claims 1 and 2, wherein the distal portion includes at least two rods.

6. The urinary catheter of any of the preceding claims, wherein the proximal portion has a proximal end that comprises a nelaton, curved, tapered or straight shape.

7. The urinary catheter of any of the preceding claims, wherein the catheter is formed by injection molding.

8. The urinary catheter of any of the preceding claims, further including a hydrophilic coating on the catheter shaft.

9. The urinary catheter of claim 8, wherein the hydrophilic coating is on the outer surface of the catheter shaft.

10. The urinary catheter of claim 8 or claim 9, wherein the hydrophilic coating is on the inner surface of the catheter shaft.

11. The urinary catheter of any of the preceding claims, wherein the catheter shaft has a gel on the outer catheter surface to aid insertion.

12. The urinary catheter of any one of the preceding claims, further including a handle (60, 160, 261) associated with the distal portion of the catheter shaft.

13. The urinary catheter of claim 12, wherein the distal portion bends between the rod and the handle.

14. The urinary catheter of claim 12, wherein the bend is between 30° and 90°.

15. The urinary catheter of any of claims 12-14, wherein the catheter further includes at least flexible piece attached to the at least one rod and handle.

## Patentansprüche

1. Urinkatheter (10, 111, 212) mit einem Katheterschaft (15, 115, 215), der einen distalen Abschnitt (13b, 113b, 213b) und einen proximalen Abschnitt (13a, 113a, 213a) mit einer Spitze beinhaltet, die durch die Harnröhre eines Benutzers eingeführt wird;
mindestens eine Öse (40a, 140a, 241a, 40b, 140b, 241b) im proximalen Abschnitt; wobei die mindestens eine Öse in Kommunikation mit einem inneren Lumen (100, 201) des proximalen Abschnitts steht, um Urin aus der Blase abzuleiten; mindestens eine Drainageöffnung (50, 150, 246) im Katheterschaft zum Ableiten von Fluid aus dem inneren Lumen, wobei die mindestens eine Drainageöffnung zwischen dem proximalen Abschnitt und dem distalen Abschnitt des Katheterschafts liegt, derart, dass während der Katheterisierung die Drainageöffnung konfiguriert ist, um sich innerhalb der Harnröhre zu befinden;
der distale Abschnitt, der mindestens eine Stange (30, 130, 232a, 232b) beinhaltet; und
die mindestens eine Drainageöffnung zum Ableiten von Fluid aus dem inneren Lumen zur Außenseite der mindestens einen Stange des distalen Abschnitts des Katheterschafts;
**dadurch gekennzeichnet, dass** eine Querschnittsbreite der mindestens einen Stange kleiner ist als eine Querschnittsbreite des proximalen Abschnitts.

2. Urinkatheter nach Anspruch 1, wobei die mindestens eine Stange eine feste Konstruktion ist.

3. Urinkatheter nach einem der Ansprüche 1 und 2, ferner beinhaltend eine Verengung zwischen dem proximalen Abschnitt und der Stange.

4. Urinkatheter nach Anspruch 3, wobei die Verengung eine partielle Verengung oder eine Umfangsverengung ist.

5. Urinkatheter nach einem der Ansprüche 1 und 2, wobei der distale Abschnitt mindestens zwei Stangen beinhaltet.

6. Urinkatheter nach einem der vorhergehenden Ansprüche, wobei der proximale Abschnitt ein proximales Ende aufweist, das eine Nelaton-, gekrümmte, konische oder gerade Form umfasst.

7. Urinkatheter nach einem der vorhergehenden Ansprüche, wobei der Katheter durch Spritzgießen gebildet wird.

8. Urinkatheter nach einem der vorhergehenden Ansprüche, ferner beinhaltend eine hydrophile Beschichtung auf dem Katheterschaft.

9. Urinkatheter nach Anspruch 8, wobei die hydrophile Beschichtung auf der Außenfläche des Katheterschafts ist.

10. Urinkatheter nach Anspruch 8 oder Anspruch 9, wobei die hydrophile Beschichtung auf der Innenfläche des Katheterschafts ist.

11. Urinkatheter nach einem der vorhergehenden Ansprüche, wobei der Katheterschaft ein Gel auf der äußeren Katheteroberfläche aufweist, um das Einführen zu erleichtern.

12. Urinkatheter nach einem der vorhergehenden Ansprüche, ferner beinhaltend einen Griff (60, 160, 261), der dem distalen Abschnitt des Katheterschafts zugeordnet ist.

13. Urinkatheter nach Anspruch 12, wobei sich der distale Abschnitt zwischen der Stange und dem Griff biegt.

14. Urinkatheter nach Anspruch 12, wobei die Biegung zwischen 30° und 90° liegt.

15. Urinkatheter nach einem der Ansprüche 12-14, wobei der Katheter ferner mindestens ein flexibles Stück beinhaltet, das an der mindestens einen Stange und dem Griff befestigt ist.

## Revendications

1. Cathéter urinaire (10, 111, 212) comportant une tige du cathéter (15, 115, 215) comportant une partie distale (13b, 113b, 213b) et une partie proximale (13a, 113a, 213a) présentant une pointe qui est insérée à travers l'urètre d'un utilisateur ;
au moins un œillet (40a, 140a, 241a, 40b, 140b, 241b) dans la partie proximale ; l'au moins un œillet étant en communication avec une lumière interne (100, 201) de la partie proximale pour drainer l'urine de la vessie ;
au moins une ouverture de drainage (50, 150, 246) dans la tige du cathéter destinée à drainer le fluide depuis la lumière interne, dans lequel au moins une ouverture de drainage se trouve entre la partie proximale et la partie distale de la tige du cathéter de telle sorte que pendant le cathétérisme, l'ouverture de drainage est configurée pour être située à l'intérieur de l'urètre ;
la partie distale comportant au moins un mandrin (30, 130, 232a, 232b) ; et
au moins une ouverture de drainage destinée à drainer le fluide depuis la lumière interne vers l'extérieur de l'au moins un mandrin de la partie distale de la tige du cathéter ; **caractérisé en ce qu'**une largeur de section transversale de l'au moins un mandrin est inférieure à une largeur de section transversale de la partie proximale.

2. Cathéter urinaire selon la revendication 1, dans lequel l'au moins un mandrin est une structure solide.

3. Cathéter urinaire selon l'une quelconque des revendications 1 et 2, comportant également un rétrécissement entre la partie proximale et le mandrin.

4. Cathéter urinaire selon la revendication 3, dans lequel le rétrécissement est un rétrécissement partiel ou un rétrécissement circonférentiel.

5. Cathéter urinaire selon l'une quelconque des revendications 1 et 2, dans lequel la partie distale comporte au moins deux mandrins.

6. Cathéter urinaire selon l'une quelconque des revendications précédentes, dans lequel la partie proximale présente une extrémité proximale qui comprend une forme de type Nelaton, courbée, conique ou droite.

7. Cathéter urinaire selon l'une quelconque des revendications précédentes, dans lequel le cathéter est formé par moulage par injection.

8. Cathéter urinaire selon l'une quelconque des revendications précédentes, comportant en outre un revêtement hydrophile sur la tige du cathéter.

9. Cathéter urinaire selon la revendication 8, dans lequel le revêtement hydrophile se trouve sur la surface externe de la tige du cathéter.

10. Cathéter urinaire selon la revendication 8 ou la revendication 9, dans lequel le revêtement hydrophile se trouve sur la surface interne de la tige du cathéter.

11. Cathéter urinaire selon l'une quelconque des revendications précédentes, dans lequel la tige du cathéter présente un gel sur la surface externe du cathéter pour faciliter l'insertion.

12. Cathéter urinaire selon l'une quelconque des revendications précédentes, comportant également une poignée (60, 160, 261) associée à la partie distale de la tige du cathéter.

13. Cathéter urinaire selon la revendication 12, dans lequel la partie distale se plie entre le mandrin et la poignée.

14. Cathéter urinaire selon la revendication 12, dans lequel la courbure est comprise entre 30° et 90°.

15. Cathéter urinaire selon l'une quelconque des revendications 12 à 14, dans lequel le cathéter comporte également au moins une pièce flexible fixée à l'au moins un mandrin et à la poignée.
